# EUROPEAN PATENT SPECIFICATION

(11) **EP 3 352 681 B1**
(45) Date of publication and mention of the grant of the patent: **10.09.2025**
(21) Application number: 16831209.8
(22) Date of filing: 25.07.2016
(51) Int. Cl.: A61B 17/00, A61B 17/04, A61B 17/068, A61B 17/072, A61B 17/32, A61B 18/08, A61B 18/14, A61B 17/29, A61B 90/00, A61B 18/00, A61B 18/04

(54) **TISSUE REMOVAL AND CLOSURE DEVICE**
GEWEBEENTFERNUNGS- UND -VERSCHLUSSVORRICHTUNG
PRÉLÈVEMENT DE TISSU ET DISPOSITIF DE FERMETURE

(30) Priority: 24.07.2015 US 201562196785 P
(43) Date of publication of application: 01.08.2018
(73) Proprietor: Touchstone International Medical Science Co., Ltd., 215123 Suzhou (CN)
(72) Inventor: HAUSEN, Berrnard A., Redwood City, California 94061 (US); DONOHOE, Brendan M., Fairfax, California 94930 (US)
(74) Representative: Winter, Brandl - Partnerschaft mbB
(86) International application number: PCT/US2016/043925
(87) International publication number: WO 2017/019629

(56) References cited:
- EP-A1- 2 382 927
- WO-A1-02/060328
- WO-A1-2015/153324
- US-A- 4 610 383
- US-A1- 2001 045 442
- US-A1- 2002 198 539
- US-A1- 2007 175 955
- US-A1- 2010 072 251
- US-A1- 2014 276 968

## Description

### FIELD OF THE INVENTIONS

The present invention relates to surgical instruments, useful in endoscopic, laparoscopic and/or open surgical procedures to effectively remove a suspect region of tissue such as a polyp, abnormal growth, cyst, tumor, lesion, or other abnormality from a base tissue structure.

### BACKGROUND

Minimally invasive surgical procedures are becoming more common as surgeons use a variety of techniques to operate with reduced trauma to the body than with open surgeries. In general, minimally invasive surgical procedures are safer than open surgeries and allow the patient to recover faster and heal with less pain and scarring. Minimally invasive surgical procedures are typically performed with one or more incisions to the body where surgical instruments are inserted and maneuvered into the surgical site to treat the targeted tissue.

A polyp is an abnormal growth of tissue projecting from a base region of tissue, typically that includes a mucous membrane. Polyps can be attached to the surface of the mucous membrane by a narrow elongated stalk, in which case the polyp is said to be pedunculated. If the polyp is directly attached to the base region of tissue with no stalk, the polyp is said to be sessile. Polyps can be found in various regions within the body such as the colon, stomach, nose, urinary bladder, cervix, small intestines, and uterus.

Polypectomy performed via an endoscopic or laparoscopic procedure performed through the oral or anal cavities are much more preferred over open procedures. Polyps are conventionally removed using either electrical forceps or an electrosurgical wire loop that is positioned around the base of the stalk of the polyp where the device severs the polyp (or stalk) as well as coagulates the blood vessels in the stalk of the polyp. However, in any procedure using conventional devices, especially those performed minimally invasively, the physician must take care to remove the polyp or stalk while remaining safely spaced away from the base region of tissue. Otherwise, inadvertent removal of the base tissue can create additional trauma via an opening in the base tissue. For example, unintentionally creating an opening in an organ, including but not limited to, the colon, stomach, nasal cavity, urinary bladder, cervix, small intestines, or uterus can require immediate surgery to repair the opening. Moreover, repairing any opening created as a result of removal of suspect tissue can require complicated or time consuming stitching or suturing to close the opening in the base tissue of the organ. There is also a risk of post-surgical complications if the physician does not properly close the opening.

Many times polyps or other suspect regions of tissue are identified during examination procedure as opposed to a surgical procedure. The risks associated with inadvertently creating an opening in an organ during removal of suspect tissue can present a dilemma for a physician that first observes the polyp (or other suspect tissue) but is hesitant to remove it if the physician believes that it will be difficult to maintain a clean margin (i.e., a region of relatively healthy tissue) at the site of the excision. In such a case, the physician must refer the patient for a further surgical procedure to remove the suspect region of tissue for a biopsy.

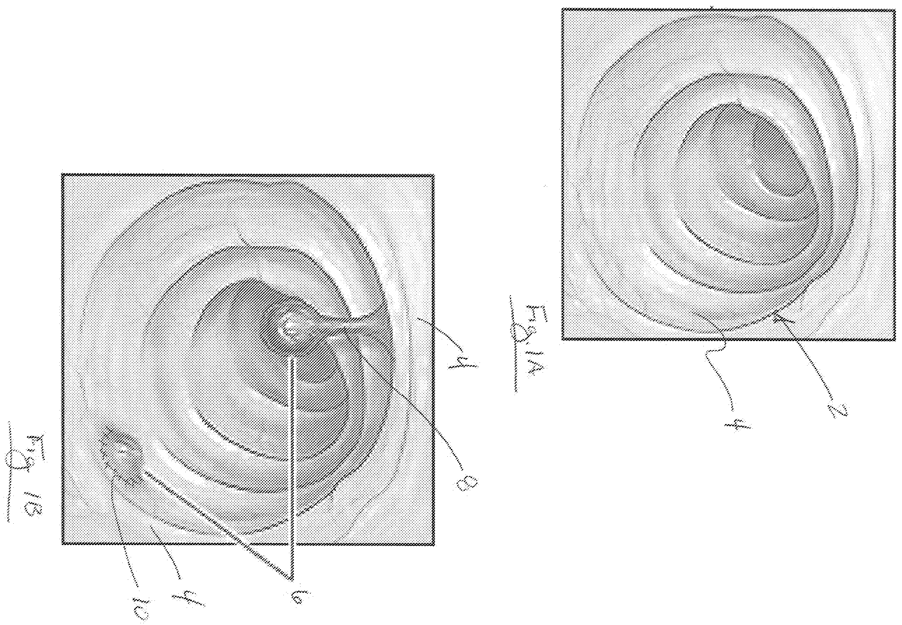

FIGS. 1A and 1B assist in illustrating the issues presented above. For example, FIG. 1A illustrates an endoscopic view (e.g., a view of a cavity or organ using endoscopic visualization) of the base tissue 4 within an organ 2. As shown, because the initial examinations are often performed via a minimally invasive procedure, the limited area or tortuosity within the organ 2 increases the difficulty of navigating and manipulating tools to the site for removal of tissue and/or closing of openings within the tissue **4.**

FIG. 1B illustrates two different polyps 6, where the polyp 6 on the upper portion of the figure connects to the base tissue 4 via a stalk 8. The polyp 6 on the lower portion of the figure directly extends from the base tissue 4 and contains a margin 10, which represents the transition of the polyp or suspect tissue 6 to the base tissue 4. In either case, a physician will only remove the polyp if the physician can be assured of removing the entire polyp without leaving suspect tissue behind. In addition, the physician will often be hesitant to remove such a region of tissue if there is a risk that the procedure creates an opening in the wall of an organ where such an opening could lead to further complications to the patient.
Related prior art is disclosed in WO 2015/153324 A1, disclosing end effectors, surgical stapling devices and methods of using same.
Further of prior art is disclosed in WO 02/060328 A1 which discloses an apparatus and a method for stapling and resectioning gastro-esophageal tissue, and in EP 2 382 927 A1 which discloses devices for placement of partitions within a hollow body organ.

There remains a need for a device, methods, and systems to allow a physician to access difficult to reach anatomic regions and to remove a region of suspect tissue in its entirety, and to provide the physician with the ability to confidently secure the tissue that remains after the excision of suspect tissue.

### SUMMARY OF THE INVENTION

Variations of the devices, methods and procedures described herein include combinations of features of the various embodiments or combination of the embodiments themselves wherever possible. The present invention is set out in the appended claim set.

The present disclosure includes surgical devices for separating a region of tissue from a tissue structure and for use with a plurality of fasteners that can fasten the region of tissue adjacent to the separated tissue. The present disclosure also relates to devices and methods for the removal of internal tissue and, more particularly tissue removal and closure devices and methods of use. The devices and method described herein can be used to remove a suspect region of tissue, including but not limited to, a polyp, abnormal growth, cyst, tumor, lesion, or other abnormality that is found on a base region of tissue. The present disclosure describes the methods and procedures with respect to polyps but are not limited to such.

In one example, such a surgical device includes a shaft; a first jaw located at an end of the shaft, the first jaw having a first end and a second end and a working surface that extends along a length of the first jaw between the first end and the second end; a second jaw being coupled to the first jaw at the first end to allow the second jaw to move relative to the first jaw between an open position and a closed position; a boundary member operatively coupling the second jaw to the first jaw at the second end such that a distal end of the boundary member moves with the second jaw between the open and closed position while remaining engaged with the first jaw, wherein application of a tensile force on the boundary member retracts the boundary member to pull the second jaw towards the closed position; where the operatively coupling of boundary member to the first jaw and second jaw also forms a mechanical barrier at the second end that prevents the tissue structure from extending longitudinally beyond the working surface when the tissue structure is positioned transversely across the working surface between the first jaw and second jaw when the second jaw is in the closed position; a fastening track comprising a plurality of fastening openings located on the working surface to permit movement of the plurality of fasteners therethrough, the fastening track being adjacent to a first edge of the first jaw to permit movement of the fasteners therethrough; a cutting track adjacent to a second edge of the first jaw; and a cutting element moveable along the cutting track, where actuation of the cutting element along the cutting tract separates the region of tissue from the tissue structure secured by the second jaw in the closed position. In some variations, the first jaw is fixed at the end of the shaft such that the second jaw moves and the first jaw remains stationary when actuated.

In another variation, the surgical devices can include a shaft; a first jaw located at the end of the shaft, the first jaw having a first end and a second end and a plurality of openings that extends along a length of the first jaw between the first end and the second end; an actuating jaw being coupled to the first jaw at the first end and moveable between an open position and a closed position; a boundary member having a distal end coupled to the actuating jaw and being moveably engaged through the first jaw at the second end to form a barrier at the second end of the first jaw, where the boundary member remains engaged with the first jaw as the distal end of the boundary member moves with the actuating jaw when the actuating jaw moves between the open position and the closed position, such that application of a tensile force on the boundary member pulls the actuating jaw towards the closed positon; wherein the barrier formed by the boundary member prevents the region of tissue from extending longitudinally beyond the plurality of openings in the first jaw while the actuating jaw is in the closed position when the tissue is positioned transversely to and between the first jaw and actuating jaw; where the plurality of openings comprises a cutting track extending adjacent to a plurality of fastening openings, the plurality of fastening openings configured to permit movement of the fasteners there through, where the plurality of fastening openings are adjacent to a first edge of the first jaw and where the cutting track extends adjacently to a second edge of the first jaw, where a length of the cutting track is greater than a length of the plurality of fastening openings; and a cutting element located within the cutting track and configured to cut the region of tissue secured between the first jaw and the actuating jaw when the first jaw and the actuating jaw are in the closed position.

Another variation of the device includes a shaft; a first jaw located at an end of the shaft, the first jaw having a first end and a second end and a working surface that extends along a length of the first jaw between the first end and the second end; a second jaw being coupled to the first jaw at the first end to allow the second jaw to move relative to the first jaw between an open position and a closed position; a fastening track comprising a plurality of fastening openings located on the working surface to permit movement of the plurality of fasteners there through, the fastening track being adjacent to a first edge of the first jaw to permit movement of the fasteners through; a cutting element moveable parallel to the fastening track, where actuation of the cutting element along the cutting tract separates the region of tissue from the tissue structure secured by the second jaw in the closed position; and where the cutting element further comprising a first extension slidably coupled to a portion of the first jaw and a second extension slidably coupled to a portion of the second jaw, such that as the cutting element moves along the first jaw, the first and second portion prevents the second jaw from moving away from the first jaw, where when the cutting element is in a distal position along the first jaw and in a proximal position along the first jaw, the second extension disengages from the second jaw to permit opening of the second jaw relative to the first jaw.

Yet another variation of the device includes a shaft; a first jaw located at an end of the shaft, the first jaw having a first end and a second end and a working surface that extends along a length of the first jaw between the first end and the second end; a second jaw being coupled to the first jaw at the first end to allow the second jaw to move relative to the first jaw between an open position and a closed position; a boundary member member operatively coupled to the second jaw at a second end of the second jaw and is moveable when the second jaw is in the open position to form a mechanical barrier at the second end that prevents the tissue structure from extending beyond the working surface when the tissue structure is positioned transversely across the working surface between the first jaw and second jaw when the second jaw is in the closed position; a fastening track comprising a plurality of fastening openings located on the working surface to permit movement of the plurality of fasteners there through, the fastening track being adjacent to a first edge of the first jaw to permit movement of the fasteners through; a cutting track adjacent to a second edge of the first jaw; and a cutting element moveable along the cutting track, where actuation of the cutting element along the cutting tract separates the region of tissue from the tissue structure secured by the second jaw in the closed position.

Variations of the devices can also include a boundary member that is configured to advance away from the first jaw when the second jaw is in the open position such that the distal end of the boundary member remains coupled to the second jaw and a portion of the boundary member expands away from the first jaw to an extended profile, where the extended profile permits capturing of the tissue structure beyond the working surface when the second jaw is in the open position, and where proximal movement of the boundary member direct the tissue structure towards the working surface as the second jaw assumes the closed position.

The boundary member can be releasably coupled to the second jaw to permit detachment of the first and second jaw from the shaft, and further comprising a cartridge containing the plurality of fasteners.

The jaw assembly can be spring biased to the open position or the closed position.

In variations of the device, cutting track spans beyond the fastening track on the first and second end of the fixed jaw.

The cutting element disclosed herein can comprise a mechanical instrument such as a blade, an electrosurgical cutting apparatus, or a combination of both.

The devices described herein can have any number of parallel rows of fastening openings.

### BRIEF DESCRIPTION OF DRAWINGS

FIG. 1A illustrates a view of a cavity or organ using endoscopic visualization of a base tissue within an organ.
FIG. 1B illustrates two different regions of suspect tissue.
FIG. 2 illustrates an example of a surgical device including an operative assembly that can secure, sever and fasten a region of tissue.
FIG. 3A illustrates a magnified view of the operative jaw assembly of FIG. 2 where the operative assembly is in an open configuration.
FIG. 3B shows the operative assembly of FIG. 3A when positioned in a deployment configuration where the actuating jaw is nested against the fixed jaw with little or no space there between.
FIGS. 4A to 4C shows side views of the operative jaw assembly, showing a side or edge of the fixed jaw that is adjacent to a cutting track (not shown).
FIG. 4D is a perspective view of an operative jaw assembly to illustrate the working surface of the fixed jaw.
FIGS. 5A to 5C show respective top, side, and bottom views of an actuating jaw.
FIGS. 6A to 6C show respective top, side, and bottom respective views of a fixed jaw.
FIGS. 7A to 7C illustrate additional variations of a jaw assembly where a boundary member can be configured to advance away from the fixed and/or actuating jaws to increase an area of an operative space of the device.
FIGS. 8A to 8F illustrate an example of the devices described above when used to remove a region of suspect tissue that is attached to a base tissue or a wall of a tissue structure.
FIG. 9 illustrates an alternate variation of an operative assembly where the cutting element comprises an alternate type of cutting modality.
FIGS. 10A and 10B illustrate an example of a cartridge having a plurality of fasteners where the shaft and boundary member can be disengaged from the jaws.
FIGS. 11A to 11F illustrate another variation of a surgical device where a cutting element is configured to maintain the clamping assembly in a closed position during the process of cutting tissue.
FIGS. 12A to 12F illustrate another variation of a surgical device where a cutting element is configured to maintain the clamping assembly in a closed position during the process of cutting tissue and the cutting assembly moves over an exterior surface of the device.
FIGS. 13A to 13D show a variation of the device with a boundary member that is coupled to a single jaw member.
FIG. 14 illustrates a positioning loop used in addition to the surgical assembly.

### DETAILED DESCRIPTION

Methods and devices described herein provide for separating a suspect region of tissue from a base region of tissue and securing the base region of tissue that remains after the excision of the suspect tissue. Although the following description discusses the use of mechanical fasteners and a mechanical cutting instrument, any number of fastening modes and cutting modes can be employed in accordance with the disclosure herein as long as such modes do not conflict with the teachings disclosed herein. In addition, any number of combinations of aspects of various species, or combinations of the species themselves are within the scope of this disclosure. In addition, the methods, devices and systems described herein are well suited towards endoscopic procedures, but are not limited to such procedures. The disclosure can be combined with open procedures as well.

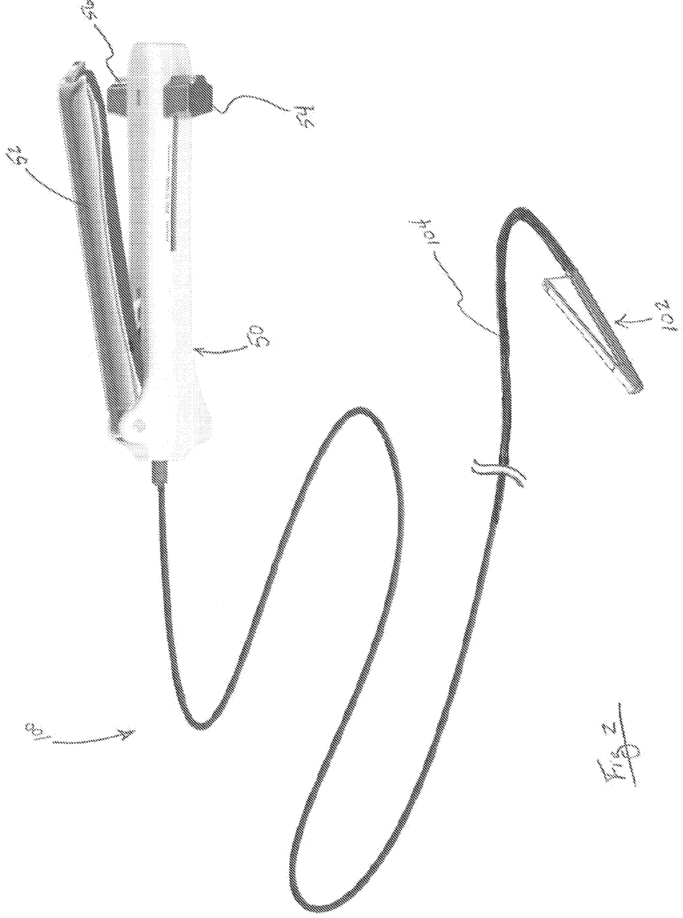

FIG. 2 illustrates an example of a surgical device **100** according to the present disclosure where the device **100** includes an operative assembly **102** that can secure, sever and fasten a region of tissue as described below. The sequence of operation of severing, securing, and fastening the region of tissue can occur in any order as desired by the user or depending upon the application. In addition, the device **100** can perform any combination of severing, securing, and fastening tissue as opposed to all of the operations.

In the variation illustrated by FIG. 2, the device **100** includes a flexible shaft **104** coupled to the operative assembly **102.** Additional variations of the shaft **104** can include a rigid, malleable, and/or steerable shaft. Furthermore, the shaft **104** can be driven via any robotic manipulator or system as required. In additional variations, the operative assembly **102** can be coupled to a distal end of an endoscope, which functions as a shaft, and where any linkages or control wires extend through the endoscope to a control assembly at the user end.

In the illustrated variation, the device **100** includes a control assembly **50** comprising a handle with various actuators, including but not limited to a clamping lever **52,** cutting actuator **54** and fastening actuator **56.** Variations of the device **100** include a control assembly **50** having an actuator that controls one or more actions of the operative assembly **102.** For example, a single lever can be configured to clamp the suspect tissue, while a second lever can be actuated to cut and fasten the respective tissue sections. In addition, as discussed below, variations of the device **100** can include a steerable operative assembly **102** or a steerable shaft **104** where the steering controls can be located on or adjacent to the handle **50.**

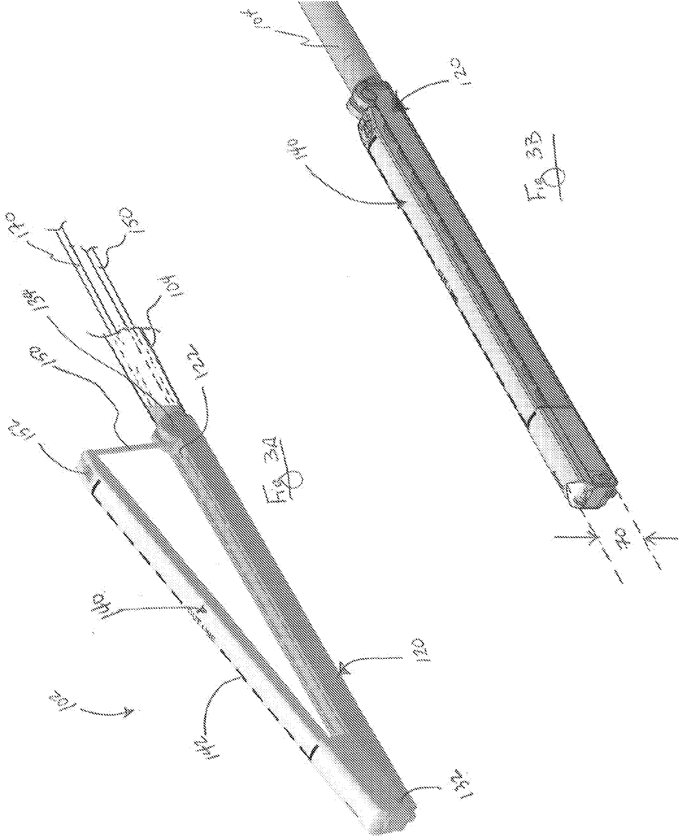

FIG. 3A illustrates a magnified view of the operative jaw assembly **102** of FIG. 2 where the operative assembly **102** is in an open configuration. In this example the assembly comprises a fixed jaw **120** coupled to a shaft **104** and where a first end of the fixed jaw **120** couples to an actuating jaw **140** via a pivot member **132.** However, any number of configurations that allow for relative movement between the jaws are within the scope of this disclosure. For example, such coupling structures can include alternate variations, such as intrinsic joints, living hinges, etc. that allow a pair of jaws to operatively secure tissue against a working surface **122.** In addition, an alternate operative jaw assembly can include a configuration where both ends of the jaw assembly move in a normal or orthogonal direction relative to a working surface **122** of one of the jaws.

As discussed below, the working surface **122** can include features necessary to perform the medical procedure. Accordingly, any portion of the operative assembly **102** can include markings **142** or visual indicators (e.g., illumination, electrodes, sensors, etc.) to assist the physician in performing the procedure. For example, marking **142** allows the physician to determine which part of tissue captured by the jaws would be adjacent to a cutting line of the device.

As noted above, the fixed jaw **120** and actuating jaw **140** function to operatively secure tissue or other structures there-between. In the variation shown, the "non-pivoting end" of the jaw assembly (i.e., the end opposite to the pivoting end) is joined with a link member **150.** The illustrated link member **150** comprises a cable, however, alternative structures can be used as a link member, including but not limited to, ribbon, filament, suture material, deformable cannula, films, or similar structure that functions as described herein. This boundary member **150** is configured to draw the jaws towards a closed configuration (as discussed below) or to a delivery configuration as shown in FIG. 3B. The boundary member **150** can include a distal end **152** that is coupled to an end of the actuating jaw **140** where a medial portion of the boundary member **150** extends through a portion of the fixed jaw and ultimately through the shaft **104.** As shown, the boundary member **150** can rely on one or more bearings or pins **134** to allow for bending of the boundary member **150.** As seen in FIG. 1, the pivoting end of the jaw assembly may be at the distal end of the operative assembly **102,** and the non-pivoting end of the jaw assembly may be at the proximal end of the operative assembly **102.** Alternately, the pivoting end of the jaw assembly may be at the proximal end of the operative assembly 102, and the non-pivoting end of the jaw assembly may be at the distal end of the operative assembly 102. FIG. 3A also shows that the operative assembly **102** can include any additional members **170** that extend through or adjacent to the shaft **104,** where the member **170** is used to actuate other features of the device (e.g., fasteners, cutting element, etc.) as described below.

In certain variations, the distal end **152** of the boundary member **150** can be detachably coupled to the actuating jaw **140** and/or the fixed jaw **120.** The boundary member **150** also provides a barrier/mechanical block/mechanical stop to prevent tissue from extending longitudinally beyond a working surface **122** of the fixed jaw **120** or a working surface (not shown) of the actuating jaw **140.**

The variation illustrated in FIG. 3A also provides an added advantage in that a tensile force or pulling force applied on the boundary member 150 or any structure coupling the boundary member 150 to the handle achieves closure of the jaw assembly. Likewise, when clamped and the boundary member is in a state of tension, the cutting element can be driven by another tensile force or pulling force applied on the linkage **170** that drives the cutting element. This configuration prevents opposite forces (e.g., a compressive and a tensile) being applied on linkages or structures in the device to clamp and then cut tissue. In use, the physician is able to apply a "pull-pull" operation to clamp and then cut the tissue.

FIG. 3B shows the operative assembly **102** of FIG. 3A when positioned in a deployment configuration where the actuating jaw **140** nests against the fixed jaw **120** with little or no space there between (in certain variations, the jaws can have a slight separation or spacing). Such a configuration minimizes a dimensional profile (e.g., a diameter, cross sectional area, height, width, etc.) of the access device or insertion point used to deliver the device **100** to the target area. As shown, the jaw height **70** is minimized in the delivery configuration. By minimizing the jaw height **70,** the operative assembly **102** is able to be introduced more easily to an operative site through a natural orifice of the patient or through an incision in the body of the patient.

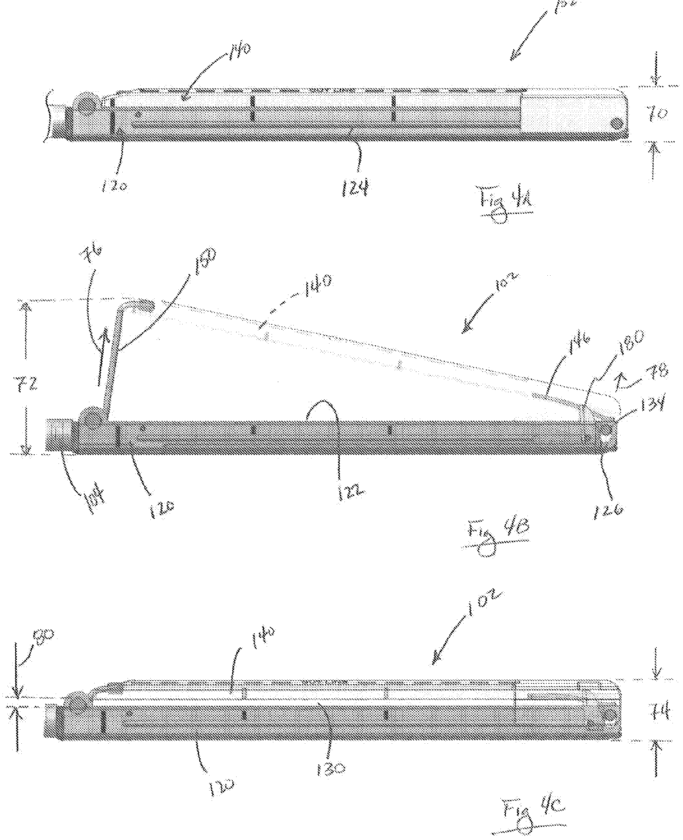

FIGS. 4A to 4C shows side views of the operative jaw assembly **102,** showing a side or edge of the fixed jaw **120** that is adjacent to a cutting track (not shown). This variation of the assembly **102** includes a window **124** in which a physician or operator can view the location of a cutting element **180** as shown in FIG. 4B. As noted above, the operative jaw assembly **102** of FIG. 4A is in a delivery configuration where the fixed jaw **140** contacts (or has a slight gap **130**) from the fixed jaw **120.**

FIG. 4B illustrates an open configuration of the operative assembly **102.** The actuating jaw **140** shown is illustrated in a hidden or transparent view for purposes of illustrating additional features of the assembly **102.** As shown, the profile of the assembly (notably the end portion adjacent to the shaft **104**) increases to an open jaw height **72** as the actuating jaw **140** and boundary member **150** move in direction **76.** FIG. 4B shows a spring member **146** that can be used to bias the actuating jaw **140** towards the open configuration. such that movement of the boundary member **150** allows the jaw to assume the open configuration.

FIG. 4B also shows an optional configuration where the fixed jaw **120** includes a slot **126** opening in that allows a pivot member **134** to move in a direction that is normal to the working surface of the jaw **122.** Such a feature allows an end of the actuating jaw **140** to pivot and move in the axial direction as noted by arrow **78** (i.e., allowing for two degrees of freedom: rotational and axial movement of the end of the jaw). The axial and rotational movement permits the operative jaw assembly **102** to assume a closed position, as shown in FIG. 4C, where the closed position allows for a space **80** between the actuating jaw **140** and the fixed jaw **120** where the space **80** accommodates tissue clamped therebetween. Additional variations of the device include configurations where the closed configuration is similar or the same to the delivery configuration where there is no space between the jaws. Alternatively, a variation of the device includes a configuration where the delivery configuration has a space between the clamping jaws.

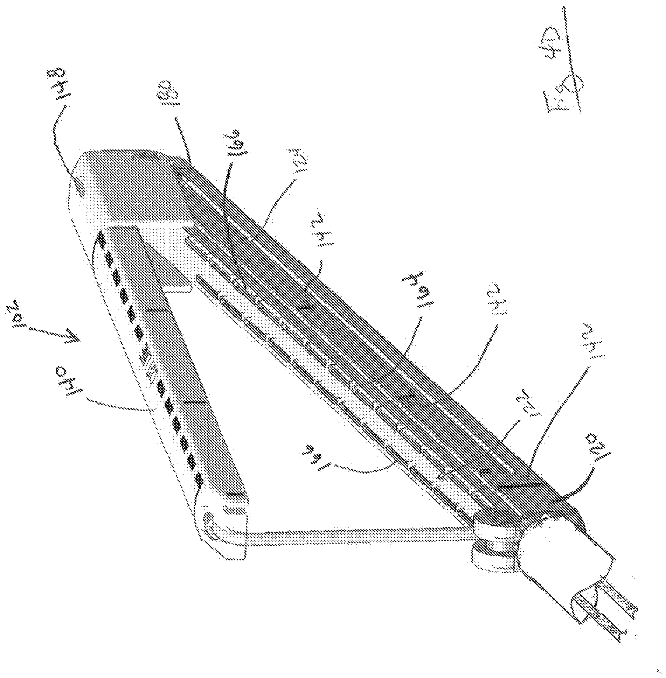

FIG. 4D is a perspective view of an operative jaw assembly **102** to better illustrate the working surface **122** of the fixed jaw. As illustrated, the working surface **122** can include a plurality of openings necessary for the jaw assembly **102** to perform various functions. For example, in the illustrated variation, the working surface comprises a fastening track comprising two rows of openings **166.** These openings allow tissue fasteners to exit the fixed jaw **120** and secure tissue (as discussed further below). In certain variations, the fasteners comprise staples. However, the device can employ any number of of tissue fixation modes (e.g., tissue fasteners, clips, adhesive, sutures, energy

The working surface **122** of FIG. 4D also includes a cutting track **164** that allows a cutting element **180** to cut through the tissue or structure secured by the clamp. In the illustrated variation, the cutting track **164** spans beyond the fastening track (i.e., is longer than the fastening track when measured longitudinally along the working surface **122**). In alternate variations, the fastening track can span beyond the cutting track **164**.As discussed below, this ensures that the cutting element **180** severs or cuts through an entire length of the tissue/structure secured within the operative jaw assembly **102,** which avoids creating a partial cut that prevents removal of the secured tissue from the site. The actuating jaw **140** is also shown to have a nesting opening **148** that can secure a cutting element **180** from moving when the jaw assembly is in the delivery configuration and/or in the open configuration. For example, when the assembly **102** is in the delivery configuration, the actuating jaw **140** moves toward the fixed jaw **120** such that a portion of the cutting element **180** is positioned within the opening **148.** This prevents inadvertent actuation of the cutting element **180.**

Variations of the device **100** can include any number of markings **142** located on an exterior of the device. In the illustrated variation, the markings **142** along the window allow a physician to observe the progress of the cutting element **180** as it moves through the cutting track **164.** In addition, the examples above show the fixed jaw as containing the cutting element and houses the fasteners. However, variations of the device and methods can include a configuration where either jaw can be the fixed/working jaw and that either the fixed or movable jaw can include the fasteners and/or cutting element.

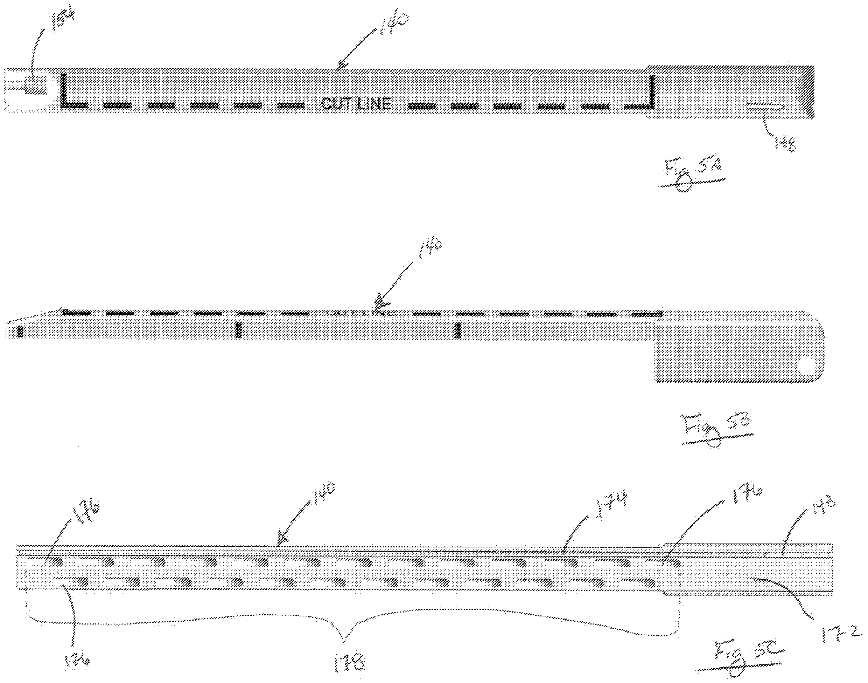

FIGS. 5A to 5C show top, side, and bottom respective views of an actuating jaw **140.** In some variations of the assembly, a boundary member can be removably coupled to an actuating member **140.** For example, as shown, FIG. 5A illustrates the actuating jaw **140** including a retaining groove **154** that allows for connection of the boundary member. Removal of the boundary member may be necessary for disassembly of the jaw assembly when loading fasteners, cleaning the device, replacing the cutting element, etc. Any number of temporary fastening structures can be used to couple the boundary member to the jaw assembly. In additional variations, the boundary member can be fixedly attached to the jaw member. FIG. 5C illustrates a working surface of the actuating jaw **140** including a cutting track **174** adjacent to an edge of the actuating jaw **140.** The cutting track **174** of the actuating jaw **140** will align with the cutting track of the fixed jaw **120** when in the delivery and/or closed position. As shown, the actuating jaw **140** can also include a fastening track **178** that contains a plurality of cavities **176.** In the example shown, the cavities **176** form two rows but any number of rows is within the scope of the disclosure. The cavities **176** can be shaped to assist in directing of the fasteners upon deployment of the fasteners in tissue. As shown, the fastening track is adjacent to an opposite edge of the fixed jaw **140** and is shorter in length than the cutting track **174** when measured along the working surface.

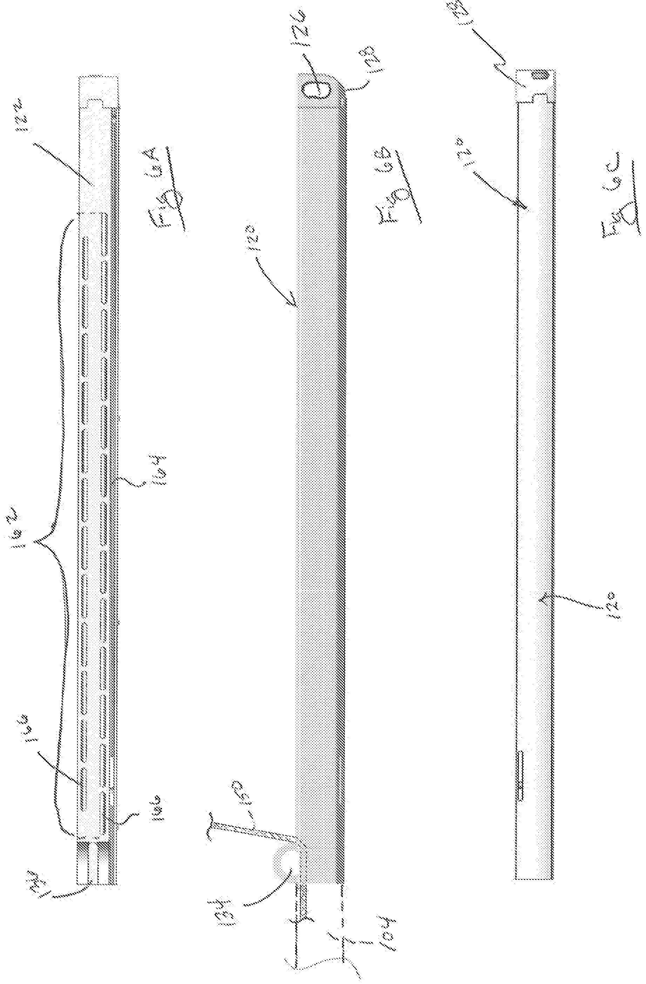

FIGS. 6A to 6C show top, side, and bottom respective views of a fixed jaw **120.** As with the variation shown in FIGS. 5A to 5C, the working surface **122** of the fixed jaw includes a plurality of openings that respectively mate with the openings on the working surface of the actuating jaw **140.** In the illustrated variation, the fastening track **162** comprises two rows of openings **166** that allow for fasteners to deploy through the working surface **122** into tissue captured within the jaw assembly. Again, any number of rows or openings can be used with the designs shown herein. In addition, for the illustrated variation, the cutting track **164** spans beyond a length of the fastening track **162.** For example, when the fastening track **162** comprises a plurality of openings containing fasteners, the length of the cutting track will span beyond the length of the jaw that contains the openings. However, alternate variations of the device can include one or more cutting tracks that extend equal to or less than the span of the fastening track. As with the actuating jaw described above, the fastening track **162** is adjacent to an edge of the fixed jaw **120** while the cutting track is adjacent to an opposite edge of the jaw **120.**

FIG. 6B also illustrates a boundary member **150** being operatively coupled to the the fixed jaw **120.** In this example, the boundary member **150** is coupled through a slot or opening **136** in the fixed jaw that resides under a pivot or bearing **134** that is coupled to the fixed jaw **120.** In alternate variations, the slot or opening **136** can extend through a body of the fixed jaw. In any case, the boundary member **150** can be coupled to the fixed jaw so that the boundary member **150** operatively couples the actuating jaw to the fixed jaw at an end of the assembly such that a distal end of the boundary member moves with the actuating jaw through the open and closed positions while remaining engaged to the fixed jaw. In the variations shown above, the boundary member is coupled to an end of the jaw that is adjacent to a shaft **104.** However, additional variations of the device can include a boundary member that tends through a length of the jaw assembly so that it operatively couples the ends of the jaw assembly farthest from the shaft. In such a case, the mouth of jaw assembly opens in a distal oriented manner as opposed to the proximal oriented manner shown in FIG. 2 and above.

FIG. 6C illustrates a bottom view of the fixed jaw assembly **120.** As shown, the jaw assembly **120** can comprise one or more components. In the illustrated example, the jaw assembly **120** includes a main body and an extension member **128** that houses the slot **126** that permits coupling of the actuating jaw to the fixed jaw **120.** Clearly, it is within those skilled in the art to construct a jaw assembly from any number of components.

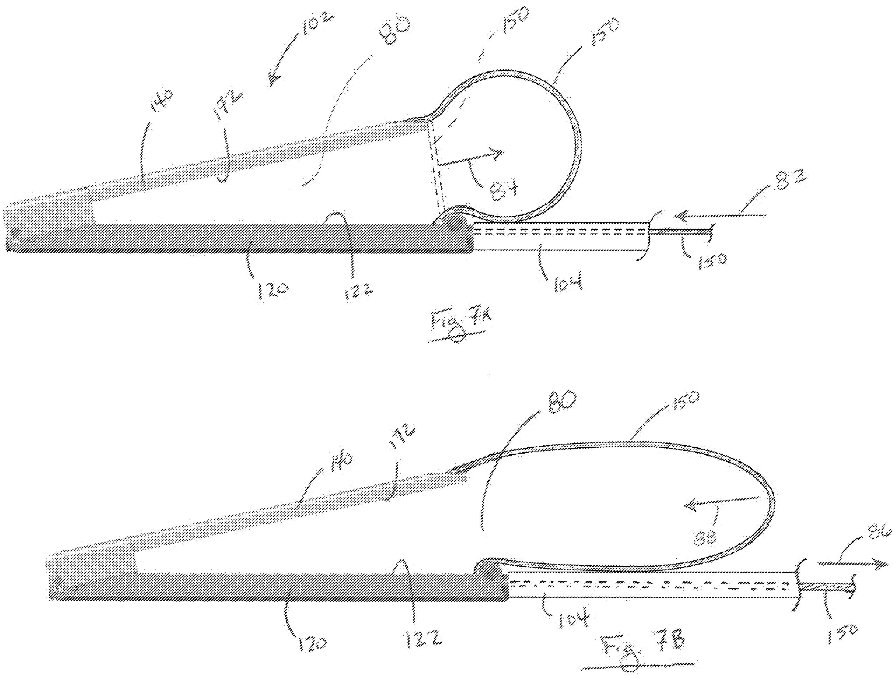

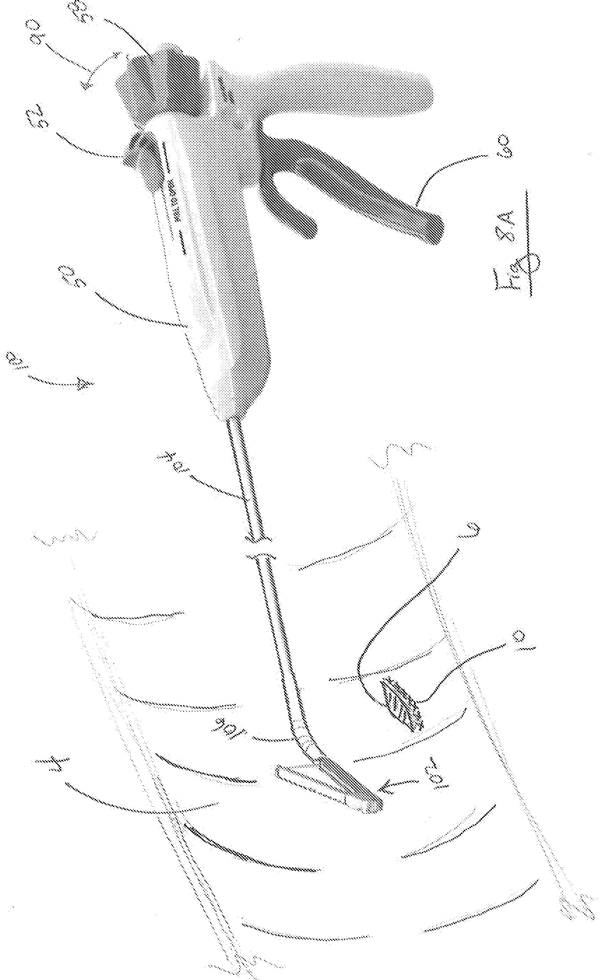

FIGS. 7A to 7C illustrate additional variations of a jaw assembly **102** where a boundary member **150** can be configured to advance away from the fixed **120** and/or actuating **140** jaws. For example, as shown in FIG. 7A, the operative jaw assembly **102** can assume a primary open configuration via, for example, spring biasing of the actuating jaw **140** relative to the fixed jaw **120** (as illustrated above). This configuration creates an operative space **80** defined by the area between the boundary member **150** (as shown by hidden lines) and jaw members **120** and **140.** The primary open configuration yields an operative space that does not extend in an axial direction beyond the working surfaces **122, 172** of the jaws **120** and **140** while still allowing tissue to be pulled across and beyond the working surface in a transverse direction (see FIGS. 8A to 8F below). However, in certain variations, a physician can actuate the device to an extended open configuration (such as shown in FIG. 7B), for example, by applying a force **82** that moves the boundary member **150** at an operative end of the device. The actuation force **82** produces movement **84** of the boundary member **150** from a primary profile (for example as denoted by the boundary member **150** configured in a straight line between jaws as shown in FIG. 7A), to an extended open configuration (such as shown in FIG. 7B) where the boundary member advances away from the jaws in direction **84.** This extended open configuration increases the operative space **80** area between the jaws and boundary member but the operative space **80** now extends beyond the working surfaces **122** and **172** of the jaw members **120** and **140.** In order to achieve the extended open configuration the boundary member **150** should have sufficient column strength such that can it does not buckle within the shaft **104** when the actuation force **82** applies a compressive force. Alternatively, or in combination, the portion of the boundary member **150** within the shaft **104** can be sized in close tolerance with a tube or passage to prevent buckling of the boundary member **150** within the shaft **104.**

This increase in operative space provides more room for a physician to maneuver larger regions of tissue into the operative space (e.g., for handling large tumors or tissue), to increase angular access to tissue, to accommodate two or more devices to grab tissue, etc. The extended open configuration can be limited to a semicircle (such as shown in FIG. 7A) or can be further extended (such as shown in FIG. 7B). Moreover, the boundary member **150** can include intrinsic joints, flex joints, varying degrees of thickness, etc., to provide a preferential shape when in the extended open configuration. Moreover, the boundary member can be shape set, and/or comprise a shape memory alloy to form any particular desired shape. Furthermore, while FIGS. 7A to 7C illustrate the boundary member **150** as extending through a length of the shaft **104,** in some variations the boundary member need not extend through the shaft. In some variations of the device can include a wire or other coupling member that is intermediate to the boundary member 150 and device controls at the operator end of the device.

FIG. 7B illustrates an elongated extended opening as a retraction force is applied to retract the boundary member **150** from the extended open configuration towards the primary open configuration. The boundary member **150** can be actuated by the operator through any number of configurations on the handle. The movement of the boundary member **150** draws or urges tissue located within the extended operative space **80** towards the working surfaces **122 172** of the jaws **120 140.** In some cases, a physician will retain tissue in the operative space **80** with the aid of a grasping device such as a forceps, clamp, suture, etc. Once the physician positions the boundary member **150** in the primary open configuration (see e.g., FIGS. 4B and 4D) the boundary member **150** and jaws **120 140** prevent any tissue located in the operative space **80** from extending beyond the working surfaces **122 172** of the jaws. The jaws **120 140** then assume the closed configuration (e.g., FIG. 4C) where a physician can continue the procedure. The ability to prevent tissue from extending longitudinally beyond the working surfaces of the jaws and to remain within the operative space **80** (see FIG. 7B) ensures that the entire length of the tissue held by the jaw members will be cut and/or fastened. Such a feature may be significant when attempting to remove a region of suspect tissue from a wall of an organ or other tissue structure. In some variations, the boundary member need not be retracted to the primary open configuration. In some variations, the boundary member may remain in a partially extended open configuration.

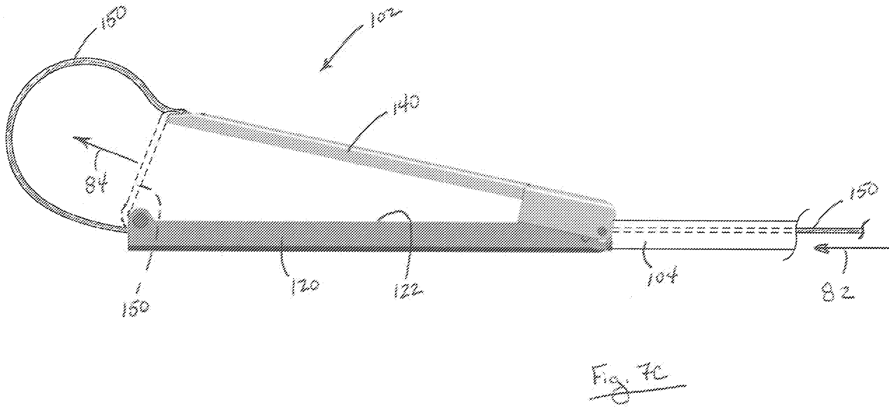

FIG. 7C illustrates another variation of an operative assembly **102** as described above, but in this example, the boundary member **150** of the operative assembly **102** is positioned on an end of the jaw members **120 140** that is opposite to a shaft **104** of the device.

FIGS. 8A to 8F illustrate an example of the devices described above when used to remove a region of suspect tissue that is attached to a base tissue or a wall of a tissue structure. As noted above, a region of suspect tissue or polyp **6** directly extends from a base region of tissue **4** (including but not limited to a wall of an organ). The suspect tissue **6** often contains a margin **10** that extends into the base tissue **4.** In some cases, the margin **10** is difficult to identify with certainty so a physician attempting to remove the suspect tissue **6** will want to remove a section of the base tissue **4** to ensure that the margins of the suspect tissue are excised.

FIG. 8A illustrates an example of a device **100** as described herein, where a physician advances an operative assembly **102** of the device **100** adjacent to a region of suspect tissue **6** located on a base region **4** or wall of an organ. In most variations, the device **100** will include controls, including but not limited to rotational/steering controls **58** where rotation **90** corresponds to rotation of an articulating section **106** of the shaft **104** that allow for rotation/steering of the operative assembly **102.** The device can also include clamping controls **52** as well as fastening and cutting controls **60.** In the illustrated variation, the cutting and fastening operations can be driven by a single control **60.** However, variations of the device allow for separate controls, such as shown above in FIG. 2.

The operative assembly **102** is also illustrated as being directly coupled to a shaft **104** of the device **100** for illustrative purposes only. In some variations, the operative assembly **102** and other features of the device **100** can be advanced through or incorporated with an endoscope type device.

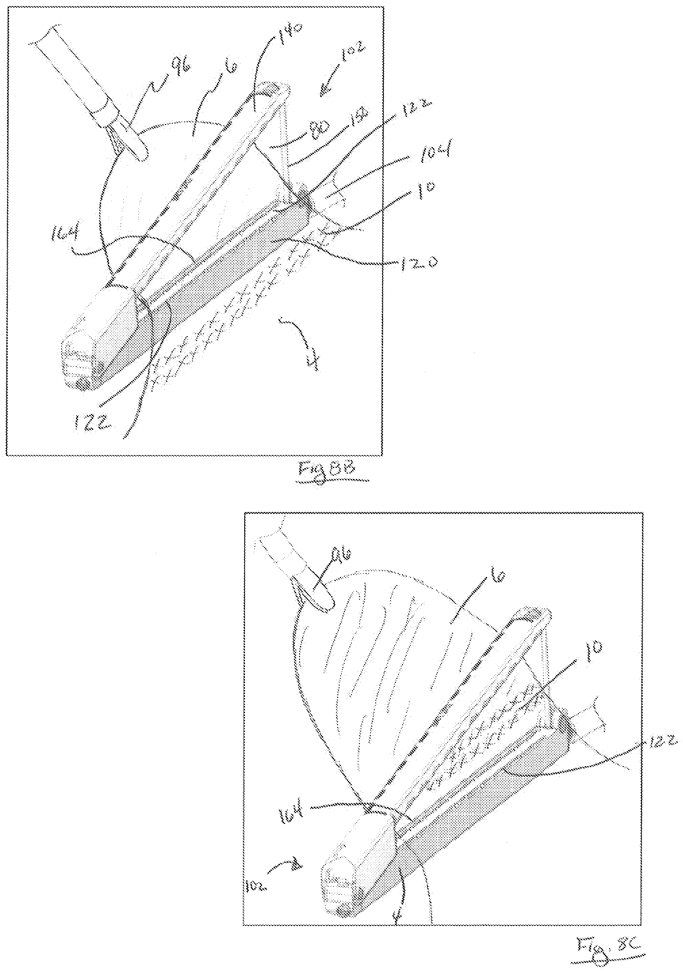

FIG. 8A illustrates a condition where the operative assembly **102** is positioned adjacent to the suspect region of tissue **6** so that a grasping device **96** can draw the suspect tissue **6** transversely across the working surface of the device, as shown in FIG. 8B. As noted above, the operative space **80** of the operative assembly **102** is bounded by the jaw members **120 140** as well as the boundary member **150.** As a result, the tissue must be drawn transversely to the operative space and/or working surface (as discussed in FIGS. 7A and 7B). In some variations where the boundary member assumes an extended open configuration, the tissue must still be drawn transversely to the jaws since the boundary member will create a closed boundary of the operative space. As shown, the suspect tissue **6** is drawn from an edge of the assembly **102** that contains the cutting track **164** across the working surface **122** and beyond the edge of the assembly **102** adjacent to the fastening track **162.** In some variations, the suspect tissue may be first drawn across the edge of the assembly 102 adjacent fastening track 162.

FIG. 8C illustrates continued movement of the suspect region of tissue **6** until the margin **10** of the suspect tissue **6** is also drawn across the working surface **122** of the assembly **102.** The physician will continue to move the tissue until a region of base tissue **4** is adjacent to the working surface **122** of the assembly **102.** Positioning the fastening track closer to the margin **10** than the cutting track assists with removing an entirety of the suspect tissue **6** while securing the adjacent base region **4.**

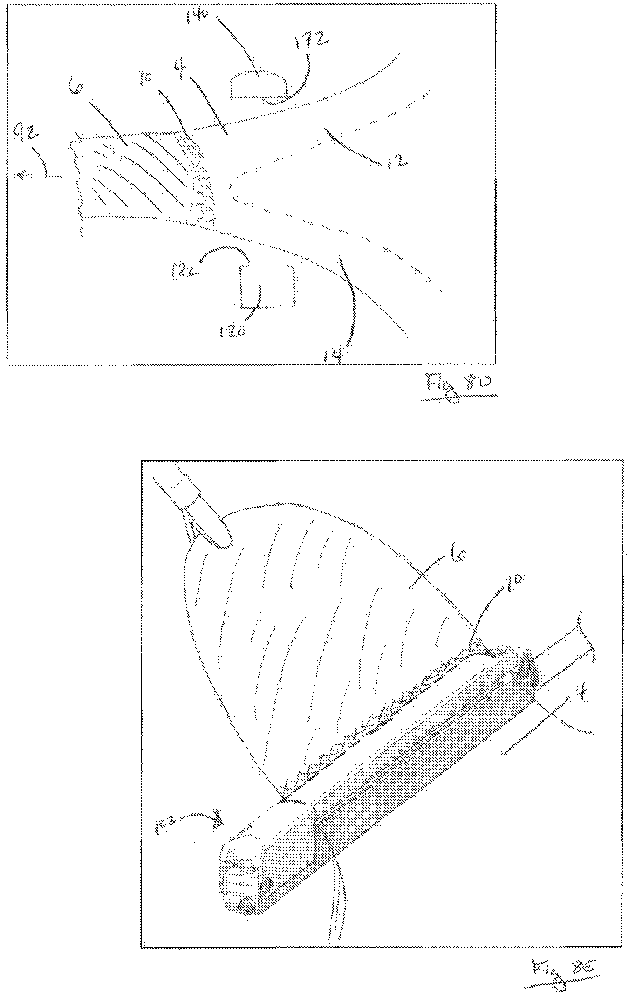

FIG. 8D illustrates a schematic side view of the suspect region of tissue **6,** margin **10** and tissue wall **4** being transversely moved across the jaw members **120 140.** As shown, pulling the wall of the tissue **4** into the operative space of the jaw causes a first region **12** and a second region **14** of the wall **4** that is adjacent to the suspect tissue **6** to become located adjacent to the working surfaces **122 172** of the jaw members **120 140.** Actuation of the jaw members **120 140** to the closed position, as shown in FIG. 8E, causes the first region **12** and second region **14** of the wall **4** to contact and become a clamped or joined region of tissue **16** (as seen in FIG. 8E). In some cases, the regions **12 14** are already joined, and the stapling prevents/minimizes bleeding through the incision line so the region is simply a clamped region with a plurality of fasteners driven through the clamped tissue.

When the operative assembly **102** secures the tissue wall **4** in the closed position, the physician can inspect the secured structure to ensure that a region of healthy or base tissue **4** is adjacent to the fastening side/edge of the operative assembly **102.** If the physician is uncertain or not satisfied, the jaw members can release the tissue and the physician can repeat the securing procedure.

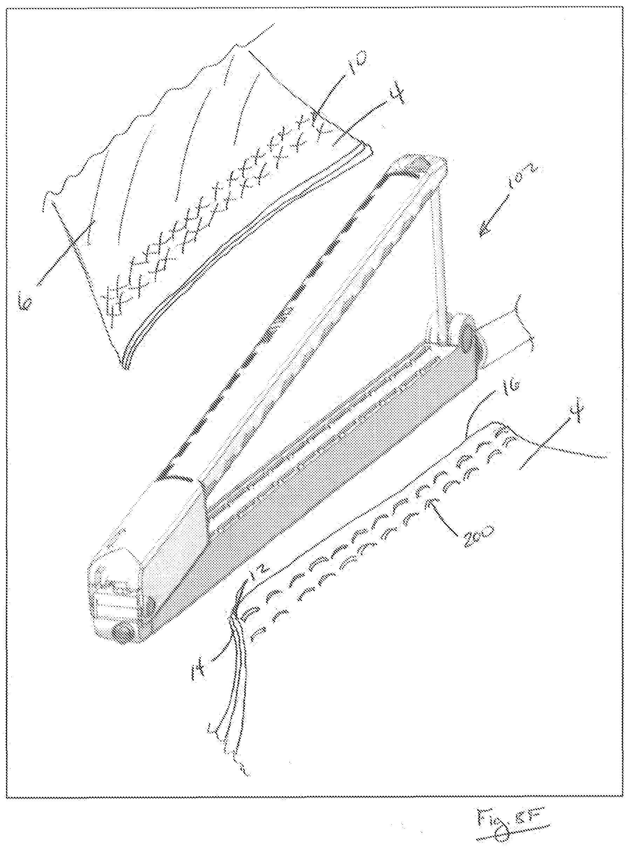

FIG. 8F illustrates the state of the procedure once the physician is satisfied with the margin of base tissue **4** and completes the cutting and fastening operations (in either order or simultaneously). FIG. 8F illustrates the operative assembly **102** in the open configuration with the joined region of tissue **16** (comprising fasteners **200** that secure the first region **12** and second region **14** of the wall 4 together). As noted above, the bounding of the operative site by the jaw members and boundary member ensures that the walls of the joined region of tissue are fully closed by the fasteners **200** and reduces the risk that an area of the joined region of tissue remains open to cause further patient complications.

In addition, the region of suspect tissue **6,** its margin **10** and an adjacent region of base tissue **4** can be removed from the operative site. The device is then removed from the operative site.

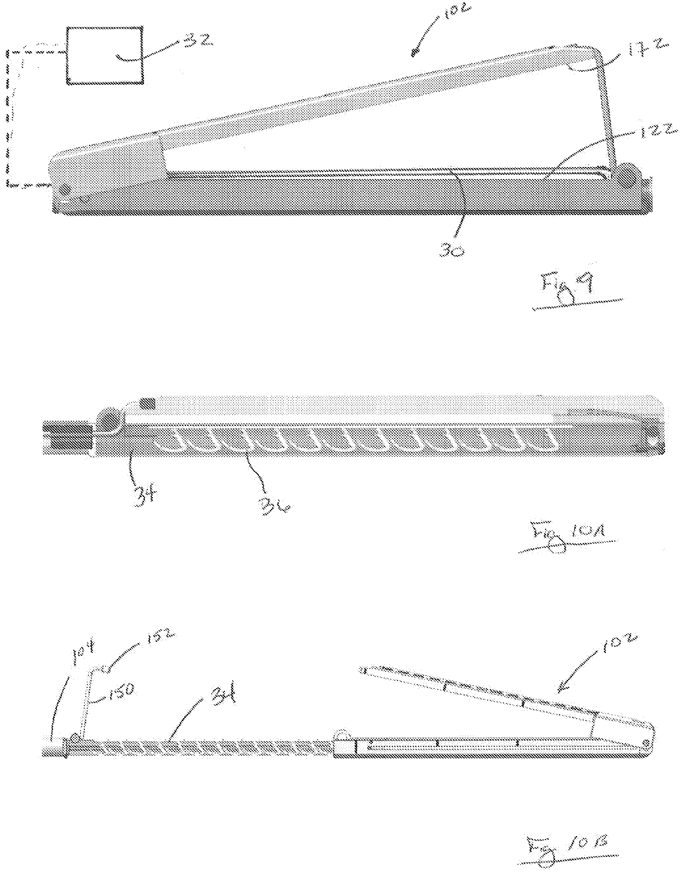

FIG. 9 illustrates an alternate variation of an operative assembly **102** where the cutting element comprises an element **30** that employs alternate modes to cut or otherwise treat the tissue, including but not limited to RF energy, resistive heat, ultrasound energy, plasma generated via a conductive fluid where the element **30** is coupleable to a power supply **32.** Alternatively, or in combination, the power supply can be coupled to one or more conductive surfaces on the working surface **122 172** of the jaws to provide a coagulation source to prevent bleeding that might occur post procedure.

FIG. 10A illustrates one example of a cartridge **34** having a plurality of fasteners **36.** Any number of fasteners can be used with the device, including but not limited to those described in US 7,988,026, US 8,403,956, US 8,985,427, US 9,004,339, US 8,056,789, US 8,631,992, US 8,261,958, or US 8,662,369. Variations of the devices include a single use operative assembly where the fasteners cannot be replaced. In some variations, a new set of fasteners can be placed on the device and be available for use. For example, the operative assembly **102** can be detached from the shaft **104** and distal end **152** of the link member **150** to permit reloading of one or more fastener cartridges **34** as shown in FIG. 10B.

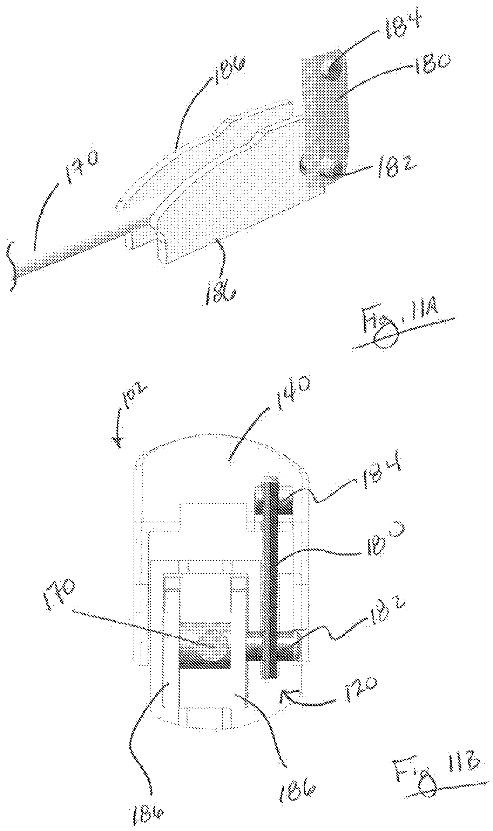

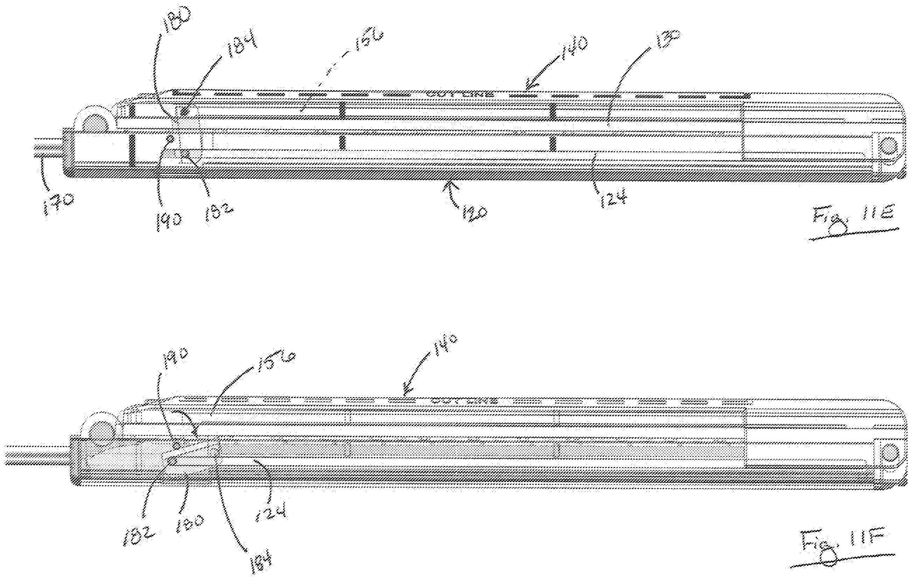

FIGS. 11A-11F illustrate another variation of a surgical device where a cutting element is configured to maintain the clamping assembly in a closed position during the process of cutting tissue.

FIG. 11A illustrates one example of a cutting element **180** affixed to one or more wedges **186** that drive the staples or fasteners from a delivery configuration through the openings in the fastening track of the jaw assembly. As disclosed in the commonly assigned patents listed above, the wedges are shaped to first pivot the fasteners through tissue and then cause the fasteners to disengage from the cartridge. In the illustrated variation, an actuator member **170** actuates both the cutting element **180** and wedges **186** so that the fastening and cutting of the tissue occurs through the same actuation. By extending the surface of the wedge **186** proximal to the cutting element **180,** fastening of tissue at a given location can occur immediately prior to cutting of the adjacent region of tissue. To ensure that the jaw assembly **102** remains closed and secures the tissue held therein during actuation, the cutting element **180** includes a first **182** and a second **184** extensions that slidably engage with the fixed jaw and second (actuating) jaw respectively. In the illustrated example, the extensions comprise pins, but any rigid structure can suffice as long as it allows slidable movement of the cutting element and while preventing separation of the jaws.

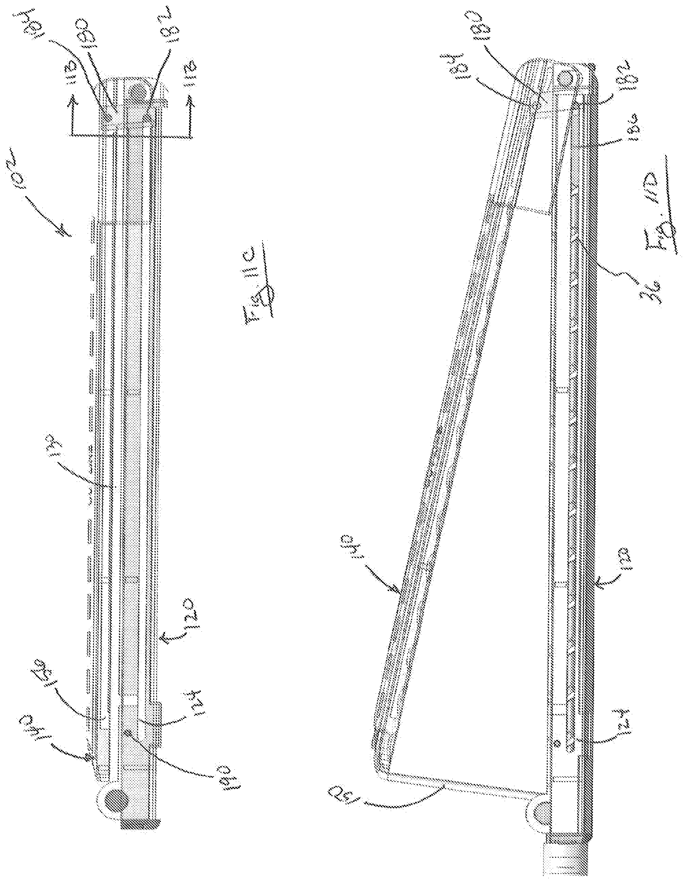

FIG. 11B illustrates a sectional view of the fixed jaw **120** and second (actuating) jaw **140** as taken along the lines 11B-11B of FIG. 11C. As illustrated, the first extension **182** engages a portion of the fixed jaw **120** while the second extension **184** engages a portion of the second jaw **140** such that the cutting element and respective extensions **182** and **184** mechanically engage the respective jaws during cutting of tissue to assist in maintaining the jaw surfaces clamped against the tissue. The extensions 184 and 182 extend laterally in both directions into grooves in the jaws as shown in FIG. 11B. The contact between the extensions **182** and **184** and grooves in the jaws holds the jaws tightly together at and near the point of cutting and fastening as the cutting element **180** and wedge 186 move along the jaws.

FIG. 11C illustrates a state of the jaw assembly **102** where the fixed **120** and actuating **140** jaws are in the closed position with a gap **130** therebetween. The cutting element **180** is located in a pre-deployment position, which in the illustrated variation is a distal most position. In this position, the first extension **182** is engaged within a slot or window **124** of the fixed jaw **120.** The second extension **184** of the cutting element **180** is not yet engaged with the actuating jaw **140** in a manner that prohibits opening of the actuating jaw **140** relative to the fixed jaw **120.** In FIG. 11C, the staples, cartridge, boundary member, and various other components are omitted for purposes of illustrating the cutting element **180** and engagement of the extensions **182, 184** with the jaw assembly.

Accordingly, as shown in FIG. 11D, the second extension **184** does not prevent the actuating jaw **140** from opening as described above. In operation, the medical practitioner will open the jaw assembly with the cutting element in the pre-deployment position. Once the physician positions tissue appropriately within the jaw surfaces, the jaw assembly clamps the tissue so that the actuating member (not shown in FIG. 11D) can withdraw the wedges **186** which drive the fasteners **36** (partially visible through the slot or window **124**) into tissue while the cutting element **180** severs tissue. Although the illustrated variation shows the cutting element as lagging to the wedges (and cuts a tissue region only after a fastener is driven into an adjacent region of tissue), variations of the device can include a cutting element leading, or even with the wedges.

FIG. 11E illustrates the cutting element **180** at the end of the cutting stroke. As shown, the second extension **184** of the cutting element moves through the track **156** in the actuating jaw **140** to engage the actuating jaw **140** and prevent opening and maximizes compression of the actuating jaw **140** relative to the fixed jaw **120.** As shown, the first extension **182** remains engaged within a slot **124** of the fixed jaw **120** during the cutting stroke. The illustrated variation of the device also shows a feature that prevents the need to retract the cutting element **180** to the pre-deployment position. Once the cutting element **180** reaches the end of the jaw assembly, cutting element **180** engages a pivot pin **190** that causes rotation of the cutting element **180** about the first extension **182** such that the second extension **184** disengages the actuating jaw **140.** Once disengaged, the actuating jaw **140** can move relative to the fixed jaw to open and release the clamped tissue.

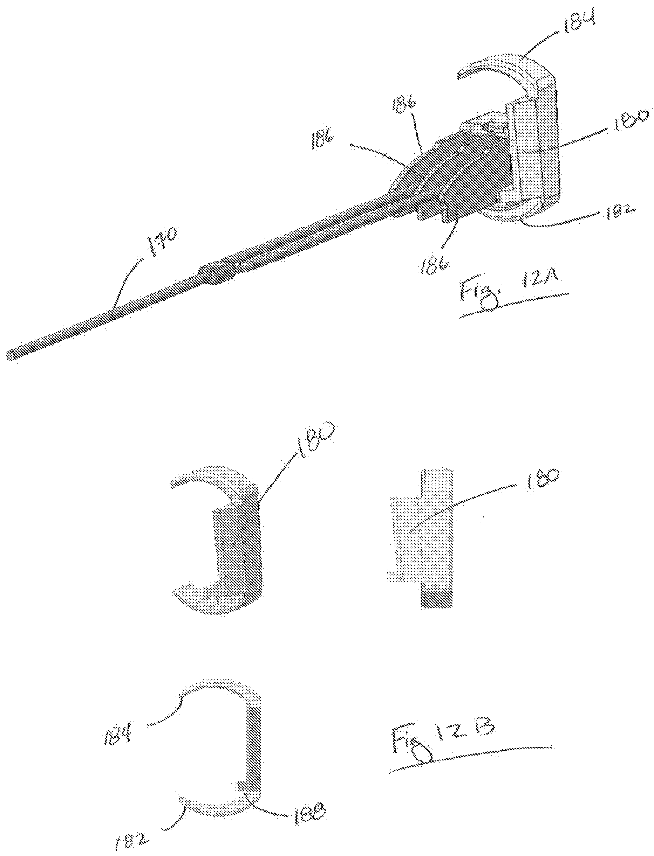

FIGS. 12A-12F illustrate another variation of a device as described herein. FIG. 12A illustrates a variation of a cutting element **180** affixed to three wedges **186** where the number of wedges corresponds to the number of fastening tracks or fastener lines in the device. While the variations illustrated in this document include 2 or 3 lines, any number of lines of fasteners are within the scope of the disclosure. The wedges **186** and cutting element can be pulled by a single actuator member **170** or multiple boundary members to allow for separate actuation of various components. In the illustrated example, the actuator member 170 bifurcates to provide an even pulling force on the wedge/cutting assembly.

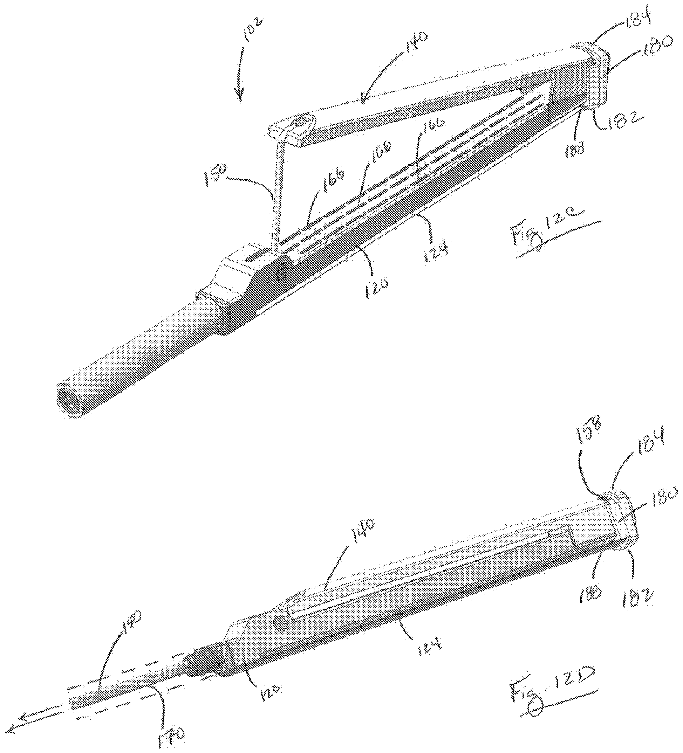

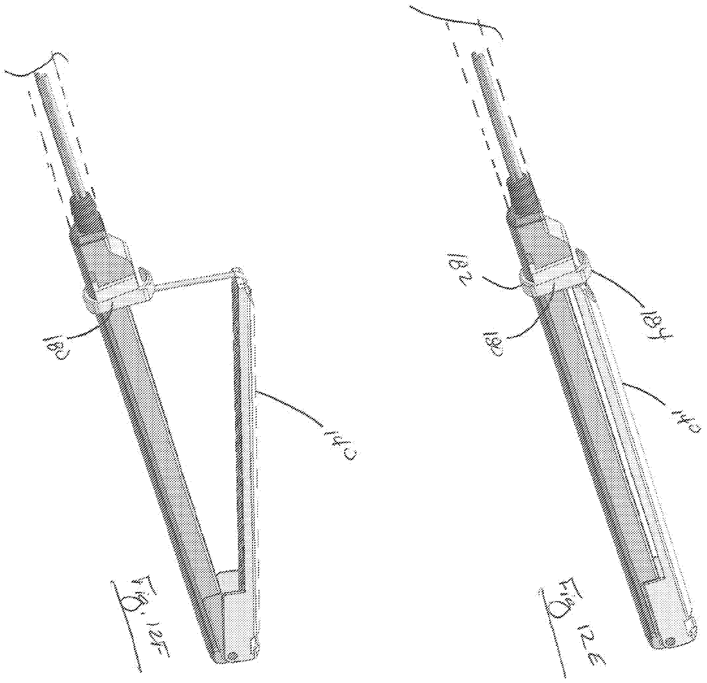

The cutting element **180** illustrates in FIG. 12A is configured to translate along an exterior side of the jaw assembly (as discussed below). However, the cutting element **180** can also include a first extension **182** and a second extension **184** to maintain closure of the jaws as the cutting element **180** moves along the jaws to cut tissue. FIG. 12B illustrates a perspective, side, and front view of the cutting element **180** and extensions **182, 184.** As discussed below, the extensions engage an exterior surface of the jaw assembly and can be shaped to match a profile of the exterior surface of the jaws. In addition, the cutting element **180** can include a key **188** that engages the fixed jaw and allow for securing of the cutting element **180** to the wedge assembly as discussed in FIGS. 12D and 12E.

FIG. 12C illustrates the cutting element **180** of FIGS. 12A and 12B on a surgical device **102.** As shown, the cutting element **180** is in a pre-deployment configuration that is located at a distal end of the jaw assembly. As such, the cutting element **180** and first extension **182** remain engaged within the fixed jaw **120** through the key **188** that extends through the slot **124** of the fixed jaw **120.** However, the second extension **184** is configured such that the actuating jaw **140** can move to an open configuration. In certain variations, the second extension **184** engages the actuating jaw **140** but to an extent that allows for opening of the jaw when the cutting element **180** is in the pre-deployment configuration. For example, in the variation shown in FIGS. 12C, the actuating jaw **140** includes a recess **188** (shown in FIG. 12D) that permits movement of the jaw. FIG. 12C also illustrates three cutting tracks **166** along a working surface of the fixed jaw **120.**

FIG. 12D illustrates the fixed **120** and actuating **140** jaws in the closed position via application of a force applied by the boundary member **150.** The actuator member **170** can then apply a force to move the cutting element **180** longa side of the jaw assembly, where the key **188** of the cutting element **180** remains engaged with the fixed jaw **120** through a slot **124** and where the first extension **182** remains engaged the fixed jaw **120** as it slides along an outer surface of the fixed jaw. The second extension **184** does not engage (or prevent movement of) the second jaw **140** until it passes the recess **158** in the second jaw **140.** Accordingly, once the cutting element **180** moves proximally along the jaw assembly, the second extension **184** engages the second jaw **140** as it slides along an outer surface of the second jaw **140.** Likewise, the cutting element **180** moves along an exerior side or lateral surface of the jaws. Therefore, as shown in FIG. 12C, this variation of the device does not include a cutting track on the jaw surface.

FIG. 12E shows the cutting element **180** in a proximal or post deployment configuration, where the second extension **184** disengages the second jaw **140.** Accordingly, the second jaw **140** can be moved into an open configuration, as shown in FIG. 12F, without movement of the cutting element **180** back to the pre-deployment configuration to the opposite end of the jaws.

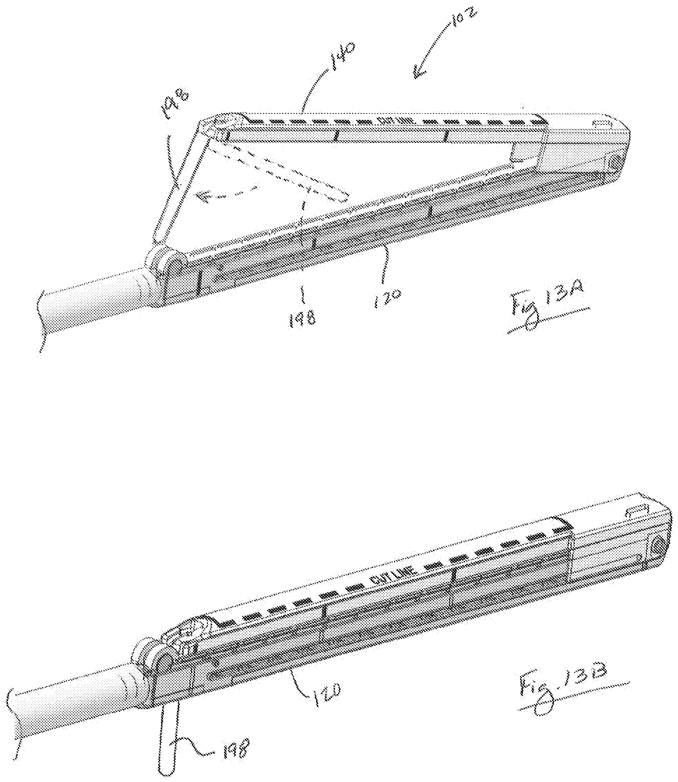

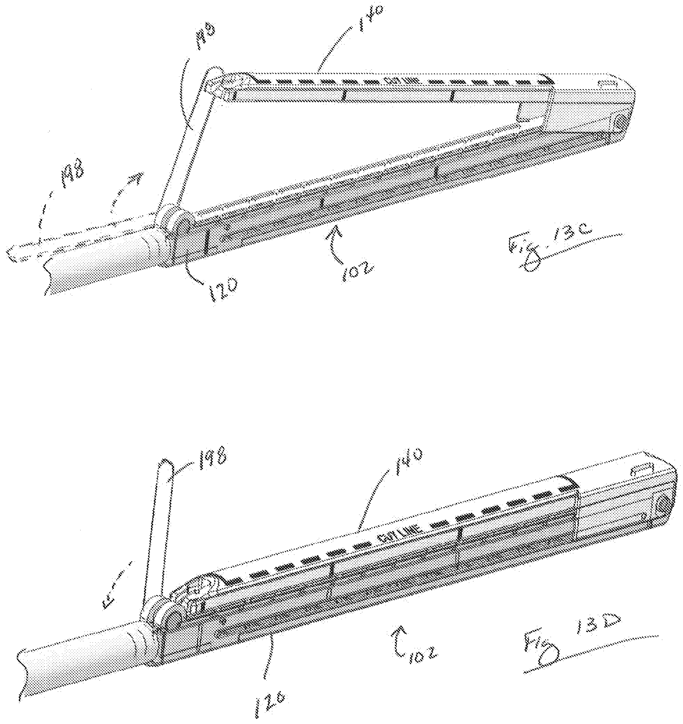

FIGS. 13A to 13D show an alternate example of a device having a boundary member **198** coupled to only one jaw member. In this variation, the boundary member **198** comprises an arm-like structure that is offset on the jaw assembly to allow pivoting to form a closed boundary. FIG. 13A shows a variation of a device **102** where the boundary member **198** is deployable from an actuating jaw **140.** As discussed above, the device **102** can be deployed with a minimum profile, where the boundary member **198** extends along a side or along a jaw surface of the actuating jaw **140.** Once the jaw opens, the physician can deploy the boundary member **198** to provide a barrier at the open side of the jaws. When the jaw is closed, as shown in FIG. 13B, the boundary member **198** can extend beyond the fixed jaw 120 or can be retracted to the actuating jaw.

FIG. 13C illustrates a boundary member **198** that is coupled to the fixed jaw 120. As noted above, the boundary member **198** can be actuated at any time to provide a barrier towards the non-pivoting of the jaws. When the jaws are closed and the procedure completed, the boundary member **198** can be retracted back to the pre-deployment configuration.

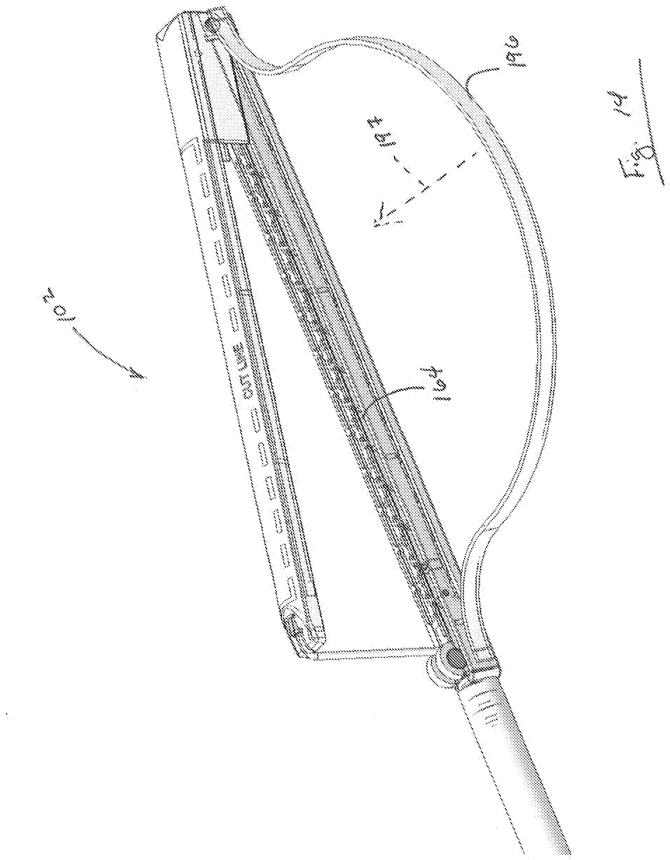

FIG. 14 shows a variation of a device **102** having a positioning loop **196** extending to a side of the device. Typically, the positioning loop extends from a side of the device adjacent to the cutting track **164.** However, alternate variations include a positioning loop adjacent to the opposite side of the device. The positioning loop **196** can be configured to be permanently bowed away from the jaw assembly. Alternatively, it can be configured to retract towards the jaw assembly (as shown by arrow **197**) when desired.

Although particular embodiments of the present invention have been described above in detail, it will be understood that this description is merely for purposes of illustration and the above description of the invention is not exhaustive. Specific features of the invention are shown in some drawings and not in others, and this is for convenience only and any feature may be combined with another in accordance with the invention. A number of variations and alternatives will be apparent to one having ordinary skills in the art. The scope of the invention is defined by the scope of the appended claim set.

## Claims

1. A surgical device (100) for separating a region of tissue from a tissue structure and for use with a plurality of fasteners, the surgical device (100) comprising:
a shaft (104);
a first jaw (120) located at an end of the shaft (104), the first jaw (120) having a first end and a second end and a working surface (122) that extends along a length of the first jaw (120) between the first end and the second end;
a second jaw (140) being coupled to the first jaw (120) at the first end to allow the second jaw to move relative to the first jaw (120) between an open position and a closed position;
a boundary member (150) operatively coupling the second jaw (140) to the first jaw (120) at the second end such that a distal end of the boundary member (150) moves with the second jaw (140) between the open and closed position while remaining engaged with to the first jaw (120), wherein application of a tensile force on the boundary member (150) retracts the boundary member (150) to pull the second jaw (140) towards the closed position;
where the operatively coupling of the boundary member (150) to the first jaw (120) and second jaw (140) also forms a mechanical barrier at the second end that prevents the tissue structure from extending in a longitudinal direction beyond the working surface (122) when the tissue structure is positioned transversely across the working surface (122) between the first jaw (120) and second jaw (140) when the second jaw (140) is in the closed position;
a fastening track (162) comprising a plurality of fastening openings (166) located on the working surface (122) to permit movement of the plurality of fasteners therethrough, the fastening track (162) being adjacent to a first longitudinal outer edge of the working surface (122) of the first jaw (120) to permit movement of the fasteners therethrough;
further comprising
a cutting track (164) and
a cutting element (180) moveable along the cutting track (164), where actuation of the cutting element (180) along the cutting track (164) separates the region of tissue from the tissue structure secured by the second jaw (140) in the closed position;
**characterized in that** the cutting track (164) is directly adjacent to a second longitudinal outer edge of the working surface (122) of the first jaw (120), wherein the second longitudinal outer edge is opposite to the first longitudinal outer edge and the cutting track (164) is closer to the second longitudinal outer edge than the first longitudinal outer edge.

2. The surgical device (100) of claim 1, wherein the first jaw (120) is fixed at the end of the shaft (104) such that the second jaw (140) moves and the first jaw (120) remains stationary when actuated, and/or wherein the boundary member (150) is configured to advance away from the first jaw (120) when the second jaw (140) is in the open position such that the distal end of the boundary member (150) remains coupled to the second jaw (140) and a portion of the boundary member (150) expands away from the first jaw (120) to an extended profile, where the extended profile permits capturing of the tissue structure beyond the working surface (122) when the second jaw (140) is in the open position, and where proximal movement of the boundary member (150) direct the tissue structure towards the working surface (122) as the second jaw (140) assumes the closed position, and/or wherein the boundary member (150) is releasably coupled to the second jaw (140) to permit detachment of the first and second jaw (140) from the shaft (104), and the surgical device (100) further comprises a cartridge containing the plurality of fasteners, and/or wherein the second jaw (140) is spring biased to the open position, and/or wherein the cutting track (164) spans beyond the fastening track (162) on the first and second end of the first jaw (120).

3. The surgical device (100) of claim 1, wherein the cutting element (180) comprises a blade, and/or wherein the cutting element (180) comprises an electrosurgical cutting apparatus, and/or wherein the fastening track (162) comprises at least two parallel rows of the plurality of fastening openings (166), and/or wherein the second end of the first jaw (120) is adjacent to the shaft (104) such that the first end of the first jaw (120) comprises a distal end of the surgical device, and/or wherein the shaft (104) comprises a flexible shaft.

4. The surgical device (100) of claim 1, further comprising a handle having an actuator, wherein the actuator is preferably coupled to the boundary member (150) and configured to actuate the boundary member (150) and/or is preferably coupled to the cutting element (180) and configured to actuate the cutting element (180).

5. The surgical device (100) of claim 1, wherein the boundary member (150) extends through the shaft (104), and/or wherein the cutting element (180) further comprises a first extension slidably coupled to a portion of the first jaw (120) and a second extension slidably coupled to a portion of the second jaw (140), such that as the cutting element (180) moves along the cutting track (164), the portion of the first jaw (120) and the portion of the second jaw (140) prevents the second jaw (140) from moving away from the first jaw (120), where the second extension disengages from the second jaw to permit opening of the second jaw (140) relative to the first jaw (120) when the cutting element (180) is either in a distal position along the first jaw (120) or in a proximal position along the first jaw (120), and/or wherein the fastening track (162) and cutting track (164) are parallel along the first jaw (120), and/or wherein the cutting element (180) is positioned to cut tissue during movement in a proximal direction, and/or wherein the first end of the first jaw (120) is located distally of the second end of the first jaw (120), and/or wherein the boundary member (150) is configured to be moved distally to assume an expanded profile when coupled to the second jaw (140) and the first jaw (120).

## Patentansprüche

1. Chirurgische Vorrichtung (100) zum Trennen eines Gewebebereichs von einer Gewebestruktur und zur Verwendung mit einer Mehrzahl von Befestigungselementen, wobei die chirurgische Vorrichtung (100) Folgendes aufweist:
einen Schaft (104);
ein erstes Maulteil (120), das an einem Ende des Schafts (104) angeordnet ist, wobei das erste Maulteil (120) ein erstes Ende und ein zweites Ende und eine Arbeitsfläche (122) aufweist, die sich entlang einer Länge des ersten Maulteils (120) zwischen dem ersten Ende und dem zweiten Ende erstreckt;
ein zweites Maulteil (140), das mit dem ersten Maulteil (120) an dem ersten Ende gekoppelt ist, um zu ermöglichen, dass sich das zweite Maulteil relativ zu dem ersten Maulteil (120) zwischen einer offenen Position und einer geschlossenen Position bewegt;
ein Begrenzungsteil (150), das das zweite Maulteil (140) operativ mit dem ersten Maulteil (120) am zweiten Ende derart koppelt, dass sich ein distales Ende des Begrenzungsteils (150) mit dem zweiten Maulteil (140) zwischen der offenen und der geschlossenen Position bewegt, während es mit dem ersten Maulteil (120) in Eingriff bleibt, wobei die Anwendung einer Zugkraft auf das Begrenzungsteil (150) das Begrenzungsteil (150) zurückzieht, um das zweite Maulteil (140) in Richtung der geschlossenen Position zu ziehen;
wobei die operative Kopplung des Begrenzungsteils (150) mit dem ersten Maulteil (120) und dem zweiten Maulteil (140) auch eine mechanische Barriere an dem zweiten Ende bildet, die verhindert, dass sich die Gewebestruktur in einer Längsrichtung über die Arbeitsfläche (122) hinaus erstreckt, wenn die Gewebestruktur quer über die Arbeitsfläche (122) zwischen dem ersten Maulteil (120) und dem zweiten Maulteil (140) positioniert ist, wenn sich das zweite Maulteil (140) in der geschlossenen Position befindet;
eine Befestigungsspur (162), die eine Mehrzahl von Befestigungsöffnungen (166) aufweist, die auf der Arbeitsfläche (122) angeordnet sind, um eine Bewegung der Mehrzahl von Befestigungselementen durch diese hindurch zu ermöglichen, wobei die Befestigungsspur (162) an eine erste Längsaußenkante der Arbeitsfläche (122) des ersten Maulteils (120) angrenzt, um eine Bewegung der Befestigungselemente durch diese hindurch zu ermöglichen;
wobei die chirurgische Vorrichtung (100) ferner Folgendes aufweist:
eine Schneidspur (164) und
ein Schneidelement (180), das entlang der Schneidspur (164) beweglich ist, wobei die Betätigung des Schneidelements (180) entlang der Schneidspur (164) den Gewebebereich von der Gewebestruktur trennt, die durch das zweite Maulteil (140) in der geschlossenen Position gesichert ist; **dadurch gekennzeichnet, dass** die Schneidspur (164) direkt an eine zweite Längsaußenkante der Arbeitsfläche (122) des ersten Maulteils (120) angrenzt, wobei die zweite Längsaußenkante der ersten Längsaußenkante gegenüberliegt und die Schneidspur (164) näher an der zweiten Längsaußenkante als an der ersten Längsaußenkante liegt.

2. Chirurgische Vorrichtung (100) nach Anspruch 1, wobei das erste Maulteil (120) am Ende des Schafts (104) derart befestigt ist, dass sich das zweite Maulteil (140) bewegt und das erste Maulteil (120) stationär bleibt, wenn es betätigt wird, und/oder wobei das Begrenzungsteil (150) so konfiguriert ist, dass es sich von dem ersten Maulteil (120) wegbewegt, wenn sich das zweite Maulteil (140) in der offenen Position befindet, so dass das distale Ende des Begrenzungsteils (150) mit dem zweiten Maulteil (140) gekoppelt bleibt und sich ein Teil des Begrenzungsteils (150) von dem ersten Maulteil (120) weg zu einem verlängerten Profil ausdehnt, wobei das verlängerte Profil ein Erfassen der Gewebestruktur über die Arbeitsfläche (122) hinaus ermöglicht, wenn sich das zweite Maulteil (140) in der offenen Position befindet, und wobei eine proximale Bewegung des Begrenzungsteils (150) die Gewebestruktur in Richtung der Arbeitsfläche (122) lenkt, wenn das zweite Maulteil (140) die geschlossene Position einnimmt, und/oder wobei das Begrenzungsteil (150) lösbar mit dem zweiten Maulteil (140) gekoppelt ist, um ein Lösen des ersten und des zweiten Maulteils (140) von dem Schaft (104) zu ermöglichen, und die chirurgische Vorrichtung (100) ferner eine Patrone aufweist, die die Mehrzahl von Befestigungselementen enthält, und/oder wobei das zweite Maulteil (140) durch eine Feder in die offene Position vorgespannt ist, und/oder wobei die Schneidspur (164) sich über die Befestigungsspur (162) am ersten und zweiten Ende des ersten Maulteils (120) hinaus erstreckt.

3. Chirurgische Vorrichtung (100) nach Anspruch 1, wobei das Schneidelement (180) eine Klinge aufweist, und/oder wobei das Schneidelement (180) einen elektrochirurgischen Schneidapparat aufweist, und/oder wobei die Befestigungsspur (162) mindestens zwei parallele Reihen der Mehrzahl von Befestigungsöffnungen (166) aufweist, und/oder wobei das zweite Ende des ersten Maulteils (120) an den Schaft (104) derart angrenzt, dass das erste Ende des ersten Maulteils (120) ein distales Ende der chirurgischen Vorrichtung aufweist, und/oder wobei der Schaft (104) einen flexiblen Schaft aufweist.

4. Chirurgische Vorrichtung (100) nach Anspruch 1 ferner mit einem Griff mit einem Betätigungselement, wobei das Betätigungselement vorzugsweise mit dem Begrenzungsteil (150) gekoppelt und so konfiguriert ist, dass es das Begrenzungsteil (150) betätigt und/oder vorzugsweise mit dem Schneidelement (180) gekoppelt und so konfiguriert ist, dass es das Schneidelement (180) betätigt.

5. Chirurgische Vorrichtung (100) nach Anspruch 1, wobei sich das Begrenzungsteil (150) durch den Schaft (104) erstreckt, und/oder wobei das Schneidelement (180) ferner eine erste Verlängerung, die gleitend mit einem Abschnitt des ersten Maulteils (120) gekoppelt ist, und eine zweite Verlängerung, die gleitend mit einem Abschnitt des zweiten Maulteils (140) gekoppelt ist, aufweist, so dass, wenn sich das Schneidelement (180) entlang der Schneidspur (164) bewegt, der Abschnitt des ersten Maulteils (120) und der Abschnitt des zweiten Maulteils (140) verhindern, dass sich das zweite Maulteil (140) von dem ersten Maulteil (120) weg bewegt, wobei die zweite Verlängerung sich von dem zweiten Maulteil löst, um ein Öffnen des zweiten Maulteils (140) relativ zum ersten Maulteil (120) zu ermöglichen, wenn sich das Schneidelement (180) entweder in einer distalen Position entlang dem ersten Maulteil (120) oder in einer proximalen Position entlang dem ersten Maulteil (120) befindet, und/oder wobei die Befestigungsspur (162) und die Schneidspur (164) entlang dem ersten Maulteil (120) parallel sind, und/oder wobei das Schneidelement (180) so positioniert ist, dass es während der Bewegung in einer proximalen Richtung Gewebe schneidet, und/oder wobei das erste Ende des ersten Maulteils (120) distal von dem zweiten Ende des ersten Maulteils (120) angeordnet ist, und/oder wobei das Begrenzungsteil (150) so konfiguriert ist, dass es distal bewegt wird, um ein erweitertes Profil anzunehmen, wenn es mit dem zweiten Maulteil (140) und dem ersten Maulteil (120) gekoppelt ist.

## Revendications

1. Dispositif chirurgical (100) pour séparer une région de tissu d'une structure de tissu et destiné à être utilisé avec une pluralité de pièces de fixation, le dispositif chirurgical (100) comprenant :
un arbre (104) ;
une première mâchoire (120) située à une extrémité de l'arbre (104), la première mâchoire (120) présentant une première extrémité et une seconde extrémité et une surface de travail (122) qui s'étend sur une longueur de la première mâchoire (120) entre la première extrémité et la seconde extrémité ;
une seconde mâchoire (140) étant couplée à la première mâchoire (120) au niveau de la première extrémité pour permettre à la seconde mâchoire de se déplacer par rapport à la première mâchoire (120) entre une position ouverte et une position fermée ;
un élément de délimitation (150) couplant de manière fonctionnelle la seconde mâchoire (140) à la première mâchoire (120) au niveau de la seconde extrémité de telle sorte qu'une extrémité distale de l'élément de délimitation (150) se déplace avec la seconde mâchoire (140) entre la position ouverte et la position fermée tout en restant en prise avec la première mâchoire (120), dans lequel une application d'une force de traction sur l'élément de délimitation (150) rétracte l'élément de délimitation (150) pour tirer la seconde mâchoire (140) vers la position fermée ;
le couplage fonctionnel de l'élément de délimitation (150) à la première mâchoire (120) et à la seconde mâchoire (140) forme également une barrière mécanique au niveau de la seconde extrémité qui empêche la structure de tissu de s'étendre dans une direction longitudinale au-delà de la surface de travail (122) lorsque la structure de tissu est positionnée transversalement à travers la surface de travail (122) entre la première mâchoire (120) et la seconde mâchoire (140) lorsque la seconde mâchoire (140) est dans la position fermée ;
une piste de fixation (162) comprenant une pluralité d'ouvertures de fixation (166) situées sur la surface de travail (122) pour permettre un déplacement de la pluralité de pièces de fixation à travers celles-ci, la piste de fixation (162) étant adjacente à un premier bord externe longitudinal de la surface de travail (122) de la première mâchoire (120) pour permettre un déplacement des pièces de fixation à travers celle-ci ;
comprenant en outre
une piste de découpe (164) et
un élément de découpe (180) pouvant se déplacer le long de la piste de coupe (164), un actionnement de l'élément de découpe (180) le long de la piste de découpe (164) séparant la région de tissu de la structure de tissu fixée par la seconde mâchoire (140) dans la position fermée ;
**caractérisé en ce que** la piste de découpe (164) est directement adjacente à un second bord externe longitudinal de la surface de travail (122) de la première mâchoire (120), dans lequel le second bord externe longitudinal est opposé au premier bord externe longitudinal et la piste de découpe (164) est plus proche du second bord externe longitudinal que du premier bord externe longitudinal.

2. Dispositif chirurgical (100) selon la revendication 1, dans lequel la première mâchoire (120) est fixée au niveau de l'extrémité de l'arbre (104) de telle sorte que la seconde mâchoire (140) se déplace et que la première mâchoire (120) reste immobile lors d'un actionnement et/ou dans lequel l'élément de délimitation (150) est configuré pour s'éloigner de la première mâchoire (120) lorsque la seconde mâchoire (140) est dans la position ouverte de telle sorte que l'extrémité distale de l'élément de délimitation (150) reste couplée à la seconde mâchoire (140) et qu'une partie de l'élément de délimitation (150) s'élargisse à l'écart de la première mâchoire (120) vers un profil étendu, le profil étendu permettant une capture de la structure de tissu au-delà de la surface de travail (122) lorsque la seconde mâchoire (140) est dans la position ouverte, et un déplacement proximal de l'élément de délimitation (150) dirige la structure de tissu vers la surface de travail (122) lorsque la seconde mâchoire (140) prend la position fermée et/ou dans lequel l'élément de délimitation (150) est couplé de manière amovible à la seconde mâchoire (140) pour permettre un détachement de la première et de la seconde mâchoire (140) à partir de l'arbre (104), et le dispositif chirurgical (100) comprend en outre une cartouche contenant la pluralité de pièces de fixation et/ou dans lequel la seconde mâchoire (140) est sollicitée par ressort vers la position ouverte et/ou dans lequel la piste de coupe (164) s'étend au-delà de la piste de fixation (162) sur la première et la seconde extrémité de la première mâchoire (120).

3. Dispositif chirurgical (100) selon la revendication 1, dans lequel l'élément de découpe (180) comprend une lame et/ou dans lequel l'élément de découpe (180) comprend un appareil de découpe électrochirurgical et/ou dans lequel la piste de fixation (162) comprend au moins deux rangées parallèles de la pluralité d'ouvertures de fixation (166) et/ou dans lequel la seconde extrémité de la première mâchoire (120) est adjacente à l'arbre (104) de telle sorte que la première extrémité de la première mâchoire (120) comprenne une extrémité distale du dispositif chirurgical et/ou dans lequel l'arbre (104) comprend un arbre flexible.

4. Dispositif chirurgical (100) selon la revendication 1, comprenant en outre une poignée présentant un actionneur, dans lequel l'actionneur est, de préférence, couplé à l'élément de délimitation (150) et configuré pour actionner l'élément de délimitation(150). et/ou est, de préférence, couplé à l'élément de découpe (180) et configuré pour actionner l'élément de découpe (180).

5. Dispositif chirurgical (100) selon la revendication 1, dans lequel l'élément de délimitation (150) s'étend à travers l'arbre (104) et/ou dans lequel l'élément de découpe (180) comprend en outre une première extension couplée de manière coulissante à une partie de la première mâchoire (120) et une seconde extension couplée de manière coulissante à une partie de la seconde mâchoire (140) de telle sorte que, au fur et à mesure que l'élément de découpe (180) se déplace le long de la piste de découpe (164), la partie de la première mâchoire (120) et la partie de la seconde mâchoire (140) empêchent la seconde mâchoire (140) de s'éloigner de la première mâchoire (120), la seconde extension se désolidarisant de la seconde mâchoire pour permettre une ouverture de la seconde mâchoire (140) par rapport à la première mâchoire (120) lorsque l'élément de découpe (180) est soit dans une position distale le long de la première mâchoire (120), soit dans une position proximale le long de la première mâchoire (120) et/ou dans lequel la piste de fixation (162) et la piste de découpe (164) sont parallèles le long de la première mâchoire (120) et/ou dans lequel l'élément de découpe (180) est positionné pour découper du tissu pendant un déplacement dans une direction proximale et/ou dans lequel la première extrémité de la première mâchoire (120) est située distalement par rapport à la seconde extrémité de la première mâchoire (120) et/ou dans lequel l'élément de délimitation (150) est configuré pour être déplacé de manière distale pour prendre un profil élargi lorsqu'il est couplé à la seconde mâchoire (140) et à la première mâchoire (120).
